# EUROPEAN PATENT SPECIFICATION

(11) **EP 2 709 549 B1**
(45) Date of publication and mention of the grant of the patent: **20.01.2016**
(21) Application number: 12726274.9
(22) Date of filing: 16.05.2012
(51) Int. Cl.: A61B 18/18, A61B 17/11

(54) **SYSTEM FOR ENERGY-BASED SEALING OF TISSUE WITH OPTICAL FEEDBACK**
SYSTEM FÜR GEWEBEVERSCHLUSS AUF ENERGIEBASIS MIT OPTISCHER RÜCKKOPPLUNG
SYSTÈME DE SOUDURE TISSULAIRE FAISANT INTERVENIR UNE SOURCE D'ÉNERGIE ET À RÉTROACTION OPTIQUE

(30) Priority: 16.05.2011 US 201113108129
(43) Date of publication of application: 26.03.2014
(73) Proprietor: Covidien LP, Mansfield, MA 02048 (US)
(72) Inventor: MISUCENKO, Igoris, Saint-Petersburg 197371 (RU); MARTSINOVSKIY, Georgy, Saint-Petersburg 194017 (RU); VERBITSKY, Mikhail, Stoughton Massachusetts 02072 (US); CHERNOV, Boris, deceased (RU)
(74) Representative: Soames, Candida Jane
(86) International application number: PCT/US2012/038112
(87) International publication number: WO 2012/158777

(56) References cited:
- WO-A1-2012/158788
- US-A- 5 336 221
- US-A- 5 470 331
- US-A- 5 762 609
- US-A- 5 769 791
- US-A1- 2005 234 437
- US-A1- 2008 221 409
- US-A1- 2010 049 187
- US-A1- 2010 217 258

## Description

### BACKGROUND

### 1. Technical Field

The present disclosure is directed to energy-based surgery and, in particular, to a system and method of employing optical feedback for energy-based tissue sealing.

### 2. Background of Related Art

Existing energy-based surgical systems and methods use electrical current or ultrasound to heat body tissue (see, for example, U.S. Pat. Nos. 7,384,420 and 7,255,697, and U.S. Patent Application Publication Nos. 2008/0039831, 2007/0173805, 2008/0147106, and 2009/0036912). In the case of electrosurgery, high-frequency electrical energy, e.g., radio frequency (RF) energy, is produced by an electrosurgical generator and applied to body tissue, e.g., vascular tissue, by an electrosurgical instrument to cut, coagulate, desiccate, and seal the tissue. The electrosurgical instrument includes electrodes that deliver the RF energy to the tissue when the electrodes physically contact the tissue.

Electrosurgical techniques and instruments can be used to coagulate small diameter blood vessels or to seal large diameter blood vessels or tissue, such as soft tissues, e.g., the lung, the brain, or the intestines. A surgeon can cauterize, coagulate, desiccate, or simply reduce or slow bleeding by controlling the intensity, frequency, and duration of the electrosurgical energy applied to the electrodes of the electrosurgical instrument and transmitted through the body tissue.

As used herein, the term "cauterization" refers to the use of heat to destroy tissue (also called "diathermy" or "electrodiathermy"). The term "coagulation" refers to a process of desiccating tissue so that the tissue cells are ruptured and dried. The term "tissue sealing" refers to the process of liquefying the collagen and elastin in tissue structures (e.g., vessels) so that they reform into a fused mass with significantly-reduced demarcation between opposing tissue structures (e.g., opposing walls of vessels). Coagulation of small vessels is usually sufficient to permanently close them. Larger vessels are sealed to assure permanent closure.

An electrosurgical surgical system typically includes a feedback control system that controls one or more parameters of the electrical energy generated by the electrosurgical generator. The feedback control system controls the electrical energy to cut, cauterize, coagulate, desiccate, or seal body tissue without causing unwanted charring of tissue at the surgical site or causing collateral damage to adjacent tissue, e.g., through the spread of thermal energy. The parameters of the electrical energy include, for example, the power, shape of the waveform, voltage, current, and/or pulse rate. Thus, the feedback control system is used to optimize the tissue-sealing process and, in particular, to provide optimal exposure to heat, to minimize thermal damage, and to reduce energy consumption.

In RF-based electrosurgical instruments, the feedback in the feedback control system is typically based on one or more electrical parameters of the tissue, such as electrical impedance of the tissue and the phase difference between voltage and current (see, e.g., U.S. Patent Application Publication Nos. 2007/0173805, 2008/0039831, and 2009/0048595). For example, commonly-owned U.S. Patent No. 6,398,779 discloses a sensor that measures the initial tissue impedance with a calibrating pulse. This initial tissue impedance is used to set various electrical parameters, e.g., current or pulse rate, by accessing a look-up table stored in a computer database. The transient pulse width associated with each calibrating pulse measured during activation is used to set the duty cycle and amplitude of the next pulse. The generation of electrosurgical power may be automatically terminated when the measured tissue impedance reaches a predetermined threshold value.

The change in tissue impedance during an electrosurgical procedure is primarily caused by the loss of water from body tissue. The water content of tissue correlates with the changes of the tissue structure during an electrosurgical procedure, e.g., a sealing procedure. However, the water content of tissue does not directly correlate with the structural changes of body tissue, such as the structural changes of the collagen and elastin, which are two major components of tissue that establish sealing bonds in tissue. Consequently, the feedback parameters based on the water content of tissue may be inaccurate, which may impact the sealing quality provided by existing electrosurgical systems and methods that use these feedback parameters.

The closest prior art is formed by document US5336221.

### SUMMARY

The invention is defined in the appended set of claims.

The surgical systems and methods of the present disclosure use optical feedback to obtain more accurate feedback parameters that are directly correlated to structural changes of tissue during a sealing procedure. As a result, the surgical systems and methods of the present disclosure provide higher sealing quality.

In one aspect, the present disclosure features a method of performing energy-based tissue sealing. The method includes illuminating tissue with light, sensing light modified by the tissue structure, analyzing the light modified by the tissue structure to determine at least one optical parameter of the tissue structure, forming a control signal based on the at least one optical parameter, generating tissue-sealing energy based on the control signal, and applying the tissue-sealing energy to the tissue.

In some embodiments, the light modified by the tissue structure may include light transmitted through the tissue structure, light reflected from the tissue structure, or light scattered by the tissue structure. Also, the at least one optical parameter may include one or more of optical transparency of the tissue structure, degree of reflection from the tissue structure, optical losses in the tissue structure caused by absorption or scattering by the tissue structure, polarization-dependent losses in the tissue structure, or degree of anisotropy of the tissue structure. Also, the control signal may control sealing energy parameters including one or more of voltage, current, pulse width, pulse frequency, amplitude, crest factor, duty cycle, repetition rate, wave shape, duration of applied sealing energy, total exposure of tissue to the sealing energy, or the spectrum of the sealing energy.

In some embodiments, the tissue-sealing energy is radio frequency (RF) energy. In other embodiments, the tissue-sealing energy is electromagnetic energy in the optical range. In yet other embodiments, the tissue-sealing energy is light and a portion of the light is used to determine the at least one optical parameter of the tissue structure.

In another aspect, the present disclosure features an energy-based tissue-sealing system. The energy-based tissue-sealing system includes an energy-based instrument, a sealing energy source coupled to the energy-based instrument, a controller coupled to the energy-based instrument, and a tissue monitor. The energy-based instrument includes a mechanism for deforming tissue and an energy applicator for applying tissue-sealing energy to the tissue. The sealing energy source generates and delivers the tissue-sealing energy to the energy-based instrument. The tissue monitor monitors at least one optical parameter of the tissue structure and the controller controls at least one parameter of the tissue-sealing energy generated by the sealing energy source based on the at least one optical parameter of the tissue structure.

In some embodiments, the at least one optical parameter of the tissue structure includes one or more of optical transparency of the tissue structure, degree of reflection from the tissue structure, optical losses in the tissue structure caused by absorption or scattering by the tissue structure, polarization-dependent losses in the tissue structure, or degree of anisotropy of the tissue structure. The tissue monitor may include a light source for illuminating the tissue with light, a sensor that senses the light modified by the tissue structure, and a processor that determines the at least one optical parameter of the tissue structure based on the light modified by the tissue structure. The wavelength of the light may be between approximately 525 nm and 585 nm.

In some embodiments, the tissue-sealing energy is light and the sealing energy source is a light source. In other embodiments, the tissue-sealing energy is ultrasonic energy and the sealing energy source is an ultrasonic generator. In yet other embodiments, the tissue-sealing energy is RF electromagnetic radiation and the sealing energy source is an RF electrosurgical generator.

The present disclosure, in yet another aspect, features an energy-based tissue-sealing instrument. The energy-based tissue-sealing instrument includes a pair of jaw members, at least one electrode, a light source, and a light sensor. The pair of jaw members are configured to deform tissue and the at least one electrode is configured to deliver tissue-sealing energy to tissue deformed by the pair of jaw members. The light source is configured to illuminate at least a portion of the tissue deformed by the pair of jaw members with light. The light sensor is configured to sense light modified by the tissue structure and to transmit a sensor signal to a controller based on the light sensed by the light sensor.

In some embodiments, the controller uses the sensor signal to determine one or more of optical transparency of the tissue, polarization-dependent losses in the tissue, or degree of anisotropy of the tissue. In some embodiments, the wavelength of the light is between approximately 525 nm and 585 nm.

In some embodiments, the tissue-sealing energy is RF electromagnetic radiation. In other embodiments, the tissue-sealing energy is light. In yet other embodiments, the tissue-sealing energy is acoustical energy.

### BRIEF DESCRIPTION OF THE DRAWINGS

Various embodiments of the present disclosure are described herein with reference to the drawings wherein:
FIG. 1 is a block diagram of an energy-based tissue-sealing system according to embodiments of the present disclosure;
FIG. 2 is a block diagram of an energy-based tissue-sealing system according to other embodiments of the present disclosure;
FIG. 3 is a block diagram illustrating a method of monitoring tissue parameters during a tissue-sealing procedure according to embodiments of the present disclosure; and
FIG. 4 is a flow diagram of a method of sealing tissue according to embodiments of the present disclosure.

### DETAILED DESCRIPTION

Embodiments of the presently disclosed system and method of employing optical feedback for energy-based tissue sealing are described in detail with reference to the drawings, in which like reference numerals designate identical or corresponding elements in each of the several views.

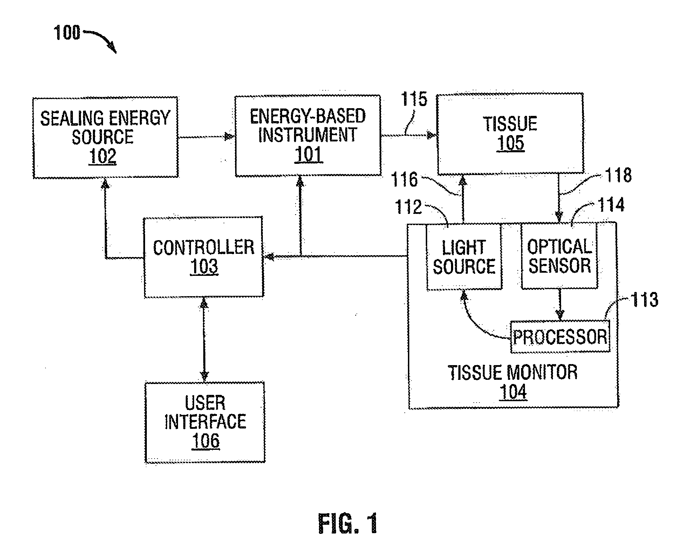

FIG. 1 is a block diagram of an energy-based tissue-sealing system 100 in accordance with embodiments of the present disclosure. The system 100 includes a sealing energy source 102, an energy-based instrument 101 for sealing tissue 105, a tissue monitor 104, a controller 103, and a user interface 106. The energy-based instrument 101 includes a mechanism for deforming the tissue 105 (e.g., jaw members that bring together opposite walls of a vessel).

The energy-based instrument 101 also includes other components that cooperate with the sealing energy source 102 to expose the tissue 105 to sealing energy 115. The controller 103 uses feedback information from the tissue monitor 104 to control parameters of the sealing energy 115 generated by the sealing energy source 102 and applied by the energy-based instrument 101 to the tissue 105.

The tissue monitor 104 includes a light source 112, a processor 113, and an optical sensor 114. Under control of the processor 113, the light source 112 of the tissue monitor 104 generates light 116 and applies it to the tissue 105. The processor 113 may control parameters of the light 116 generated by the light source 112 including the intensity, time of exposure, and polarization. The optical sensor 114 senses parameters of the light 118 reflected and/or scattered from the tissue 105, which indicates the actual optical parameters of the tissue 105. The optical sensor 114 then transmits the sensed parameters of the light 118 to the processor 113, which determines and monitors the sensed parameters of the tissue 105 based on the sensed parameters of the light 118.

Alternatively, the optical sensor 114 may transmit sensed optical parameters of the tissue 105 directly to the controller 103. The optical parameters of the tissue 105 include the optical transparency of the tissue 105, degree of reflection from the tissue 105, optical loss resulting from absorption and/or scattering by the tissue 105 (e.g., the optical polarization-dependent losses in the tissue 105), the degree of anisotropy of the optical parameters, or any combination of these optical parameters.

The controller 103 is configured to control at least one parameter of the output from the sealing energy source 102 based on the actual optical parameters of the tissue 105 sensed by the optical sensor 114. The controlled parameters of the output from the sealing energy source 102 may include one or more of the duration of applied sealing energy, the amount of applied sealing energy, the spectral parameters of the applied sealing energy, the duration of sealing pulses, or the duty cycle of sealing pulses.

In some embodiments, the energy-based tissue-sealing system 100 is integrated into a single surgical device. For example, the components of the energy-based tissue-sealing system 100 shown in FIG. 1 may be incorporated into a single portable surgical device. In other embodiments, the components of the energy-based tissue-sealing system 100 may be broken up into separate surgical instruments that are used together during a surgical procedure. For example, the system 100 may be implemented as three separate instruments: a first instrument including the sealing energy source 102 and the controller 103, a second instrument including the energy-based instrument 101 and the user interface 106, and a third instrument including the tissue monitor.

The optical sensor 114 may include a plurality of sensors for measuring a variety of tissue and energy properties (e.g., tissue transparency and polarization-dependent losses) and for providing feedback signals to the controller 103. The types of sensors and their positioning may vary for different embodiments of the present disclosure and are known to those skilled in the art.

As described in more detail below, the optical feedback signals according to the present disclosure can be used in combination with other types of feedback signals such as feedback signals from electrical or temperature sensors.

In some embodiments, the sealing energy source 102 generates optical energy or light to perform sealing procedures. A portion of this same light may be used for monitoring optical parameters of the tissue. For example, the light for monitoring optical parameters (i.e., the probing light) of tissue and the light for tissue sealing (i.e., the tissue-sealing light) may have the same frequency, but the intensity of the probing light may be less than the intensity of the tissue-sealing light. Accordingly, the energy-based tissue-sealing system may not include a separate light source 112, but a single sealing energy source 102 generating light for both tissue sealing and tissue monitoring.

In other embodiments, the energy-based system may incorporate ultrasonic technology. In these embodiments, the sealing energy source 102 is an ultrasonic generator having an ultrasonic transducer and the energy-based instrument 101 includes an ultrasonic waveguide for transmitting ultrasonic energy from the sealing energy source 102 to the tissue 105.

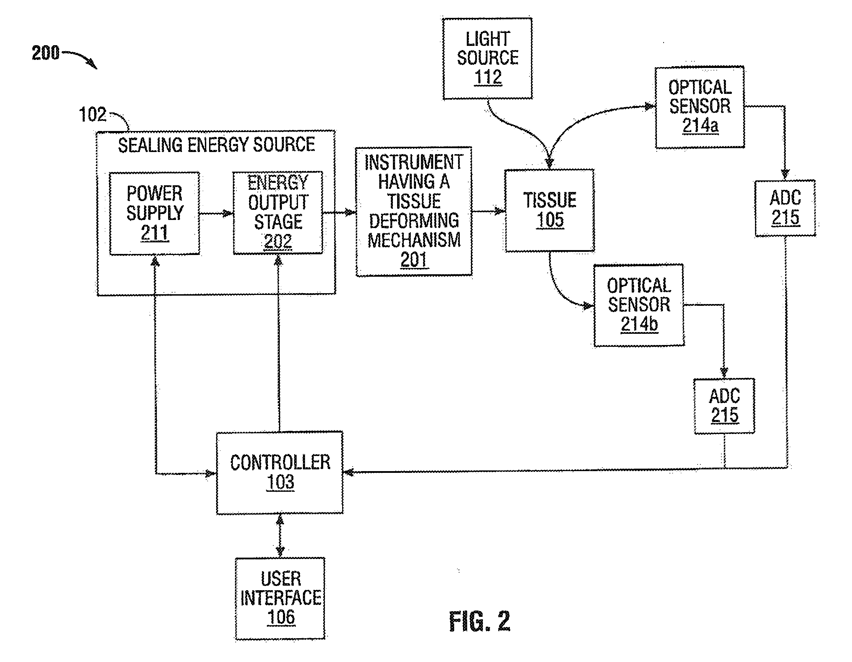

FIG. 2 is a block diagram of an electrosurgical system 200 according to other embodiments of the present disclosure. The electrosurgical system 200 includes a sealing energy source 102, an instrument having a tissue deforming mechanism 201, a light source 112, optical sensors 214a, 214b, analog-to-digital converters (ADCs) 215, a controller 103, and a user interface 106. The controller 103 is coupled to the sealing energy source 102 to control parameters of the sealing energy provided by the sealing energy source 102. The sealing energy source 102 includes a power supply 211 and an energy output stage 202. The energy output stage 202 may be configured to provide optical, electrical, or acoustical energy to the instrument having the tissue-deforming mechanism 201. The energy output stage 202 is powered by the power supply 211 and delivers sealing energy to a patient's tissue 105 via a sealing energy applicator (not explicitly shown).

In some embodiments, at least a portion of the instrument 201 is made of an optically transparent material to enable a surgeon to see the tissue 105 that is grasped by the instrument 201. Consequently, the surgeon can more easily perform visual control the grasped tissue 105. Also, the surgeon can move the instrument 201 to a greater range of positions during a surgical procedure.

In some embodiments, the energy output stage 202 is an RF-energy output stage and the sealing energy applicator includes at least one electrode. The RF-energy output stage delivers RF energy to the at least one electrode, which, in turn, applies the RF energy to the tissue. In other embodiments, the energy output stage 202 is a light-energy output stage and the sealing energy applicator includes at least one optical element, such as a waveguide and/or lens. The light-energy output stage provides light to the at least one optical element, which, in turn, directs or focuses the light on tissue.

As shown in Fig. 2, the light source 112 generates and applies light to the tissue 105 deformed by the instrument 201. Optical sensor 214a is configured to sense the light reflected from the tissue 105 and optical sensor 214b is configured to sense light transmitted through the tissue 105. The light source 112 and the optical sensors 214a, 214b may be disposed on the instrument 201 or on a separate instrument. The optical sensors 214a, 214b sense one or more optical parameters of the deformed tissue 105 and transmit this information to the controller 103, which regulates the energy output from the energy output stage 202. In particular, the optical sensors 214a, 214b provide analog sensor signals to the ADCs 215, which convert these signals to digital form and feeds them to the controller 103.

The optical sensors 214a, 214b may function together with other sensors (not shown) that sense various electrical and physical parameters or properties of the tissue 105 and communicate with the controller 103 to regulate the energy output from the energy output stage 202. The various electrical and physical parameters or properties of the tissue 105 may include: tissue impedance, changes in tissue impedance, tissue temperature, changes in tissue temperature, leakage current, applied voltage, or applied current. The other sensors may generate analog sensor signals that are provided to the controller 103 via ADCs (not shown).

The controller 103 controls the power supply 211 and/or the energy output stage 202 according to the feedback information obtained from at least one of the optical sensors 214a, 214b. The user interface 106 is coupled to the controller 103 so as to allow the user to control various parameters of the energy output from the sealing energy source 102 and applied to the tissue 105 during a surgical procedure. For example, in the case of a sealing energy source 102 that generates RF energy, the user may manually set, regulate and/or control one or more electrical parameters of the RF energy, such as voltage, current, power, frequency, intensity, and/or pulse parameters (e.g., pulse width, duty cycle, crest factor, and/or repetition rate).

The controller 103 includes at least one microprocessor (not shown) capable of executing software instructions for processing data received from the user interface 106 and the optical sensors 214a, 214b and for providing control signals to the energy output stage 202 and/or the power supply 211 based on the processed data. The software instructions are stored in an internal memory of the controller 103 and/or an external memory accessible by the controller 103, e.g., an external hard drive, floppy diskette, or CD-ROM. The digital control signals generated by the controller 103 may be converted to analog signals by a digital-to-analog converter before being provided to the sealing energy source 102.

For embodiments in which the sealing energy source 102 supplies RF energy to the tissue 105, the power supply 211 may be a high voltage DC power supply for producing electrosurgical current such as radio frequency (RF) current. Signals received from the controller 103 control the magnitude of the voltage and current output by the DC power supply. The energy output stage 202 receives the current output from the DC power supply and generates one or more pulses via a waveform generator (not shown). The pulse parameters, such as pulse width, duty cycle, crest factor, and repetition rate are regulated in response to the signals received from the controller 103. Alternatively, the power supply 211 may be an AC power supply, and the energy output stage 202 may vary the waveform of the signal received from power supply 211 to achieve a desired waveform.

The user interface 106 may be local to or remote from the controller 103. A user may enter data such as the type of surgical instrument, the type of surgical procedure, and/or the type of tissue. The user interface 106 may provide feedback to the surgeon relating to one or more parameters of the tissue 105 or other components of the surgical system 200. The user may also enter commands via the user interface 106. For embodiments in which the sealing energy source 102 supplies RF energy to the tissue 105, the commands may include a target effective voltage, current or power level, or a target response. The user may also enter commands for controlling electrical parameters of the RF energy.

The optical sensors 214a, 214b may include a wired or wireless communications interface for transmitting information to the controller 103 either directly or indirectly via the ADCs 215.

Before beginning a surgical procedure, an operator of the surgical system 200 enters information via the user interface 106. Information entered includes, for example, the type of instrument, the type of procedure (i.e., the desired surgical effect), the type of tissue, relevant patient information, and a control-mode setting. The control mode setting determines the amount of or type of control that the controller 103 will provide. At least one of the optical sensors 214a, 214b may automatically provide information to the controller 103 relating to tissue type, initial tissue thickness, initial tissue impedance, and/or other tissue parameters.

The control-mode settings may include a first mode during which the controller 103 maintains a steady selected output energy level. The control-mode settings may also include a second mode during which the controller 103 maintains a variable selected output energy level, which is a function of the time values, sensed parameters, and/or changes in sensed parameters during the surgical procedure. Operations performed on the time values and sensed parameters include operations such as calculations and/or look-up operations using a table or map stored by or accessible by the controller 103. The controller 103 processes the selected output energy values, such as by performing calculations or table look-up operations, to determine power control signal values and output control values.

The controller 103 may determine initial settings for control signals to the power supply 211 and the energy output stage 202 by using and/or processing data or settings entered by an operator, performing calculations and/ or accessing a look-up table stored by or accessible by the controller 103. Once the surgical procedure begins, the optical sensors 214a, 214b sense various optical parameters and provide feedback to the controller 103 through the ADC 215. The controller 103 processes the feedback information in accordance with the pre-selected mode, as well as any additional commands entered by the user during the surgical procedure. The controller 103 then sends control information to the power supply 211 and the energy output stage 202. The surgical system 200 may include override controls to allow the surgeon to override the control signals provided by the controller 103, if needed. For example, the surgeon can enter override commands through the user interface 106.

In some embodiments, the optical parameters of the deformed tissue are measured regardless of the area or volume of the tissue 105 that is deformed by an instrument having a mechanism for deforming tissue (e.g., jaw members). For example, the light source 112 may illuminate only a portion of the tissue 105 that is grasped between the jaw members of the energy-based instrument 101.

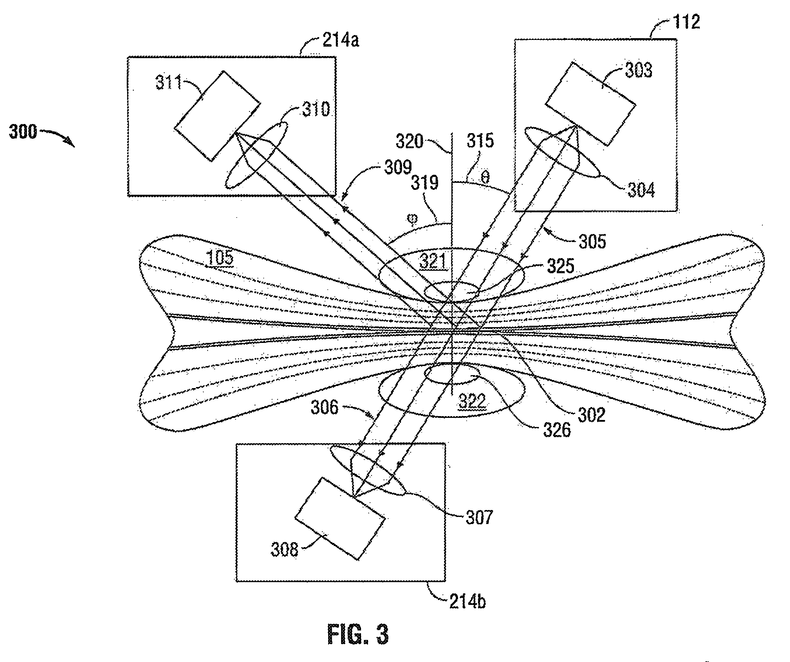

FIG. 3 is a block diagram illustrating a cross-sectional view of a general arrangement 300 for measuring optical parameters of tissue 105 during a tissue-sealing procedure according to embodiments of the present disclosure. As shown in FIG. 3, the tissue-sealing procedure involves deforming tissue 105 and a vessel 302 by grasping and compressing the vessel 302 under a predetermined pressure (3 kg/cm² to 16 kg/cm²) with the jaw members 321, 322 of an energy-based sealing instrument to bring opposite walls of the vessel 302 into contact with each other. For embodiments of the surgical system 200 that use RF energy to heat tissue, the jaw members 321, 322 include electrodes 325, 326 for applying the RF energy to the tissue 105 and the vessel 302. Thus, when the RF energy is applied to the deformed vessel 302 through the electrodes 325, 326, opposite walls of the vessel 302 are bonded together. Typically, the electrodes 325, 326 are positioned a predetermined gap distance relative to one another during the sealing process (0.001 in to about 0.006 in).

To determine the optical parameters of the tissue 105 and/or the vessel 302, the tissue 105 and/or the vessel 302 are illuminated with a light beam 305, which is generated by the light source 112. The light source 112 includes a light element 303 and a beam formation system 304. The light element 303 may be a laser, an LED, or any other similar component used to generate light. The beam formation system 304 forms a light beam 305 with appropriate spatial characteristics so that the vessel and a portion of the tissue 105 that experiences the effects of the sealing energy is exposed to the light beam 305.

In some embodiments, the beam formation system 304 includes a collimator that forms the light beam 305 by generating parallel rays of light. The angle θ 315 is the angle of the incident light beam 305 with respect to an axis 320 perpendicular to the vessel 302. The beam formation system 304 may be configured to vary the angle θ 315 of the light beam 305 over a range of angles. For example, the beam formation system 304 may select the angle θ 315 of the light beam 305 that optimizes the detection of the optical parameters of the tissue 105 or the vessel 302.

The optical parameters of the tissue 105 and vessel 302 are determined by measuring and analyzing the parameters of the light 306 transmitted through the tissue 105, which is collected by an optical system 307 and detected by a photo-sensor 308. In other embodiments, the optical parameters of the tissue 105 and vessel 302 are determined by measuring and analyzing parameters of the reflected or scattered light 309, which is collected by an optical system 310 and detected by a photo-sensor 311. The angle ϕ 319 is the angle of the reflected or scattered light beam 309 with respect to the axis 320 perpendicular to the vessel 302. The beam formation system 304 may be configured to vary the angle ϕ 319 of the light beam 309 over a range of angles. If the beam formation system 304 is configured in this way, it may select the angle ϕ 319 of the light beam 309 that optimizes the detection of the optical parameters of the tissue 105 or the vessel 302.

During the sealing process, the vessel 302 and tissue 105 are deformed and heated to change the internal structure of the vessel 302 and tissue 105. As described above, the vessel 302 and tissue 105 are compressed under a predetermined pressure to bring opposite walls of the vessel 302 into contact with each other. Then, the vessel 302 is heated, causing the restructuring of the collagen and elastin within the walls of the vessel 302. In particular, new bonds between the collagen and elastin form. These bonds stabilize when they are cooled. Heating and compressing the vessel 302 may also cause other tissue structures (e.g., organic tissue structures) to change.

The changes in the tissue structure (e.g., the restructuring of the collagen and elastin in the tissue 105), cause corresponding changes to the optical parameters of the tissue 105. These changes in the optical parameters of the vessel 302 can be detected by focusing the light beam 305 on the vessel 302 and detecting either the transmitted light beam 306 or the reflected light beam 309. The correlation between the internal structure of the vessel 302 and the optical parameters of the vessel 302 enables the surgical system 200 to control the sealing quality based on the optical parameters of the vessel 302.

Different optical properties of tissue can be detected and used as feedback information to control the sealing process. For example, optical transparency of the tissue can be a good indicator of the sealing quality. As the temperature of tissue increases during the sealing process, the collagen and elastin in the tissue melt, adjacent portions on the tissue (e.g., vessel walls) bond together, and the tissue becomes more transparent to light in the visible range of the electromagnetic spectrum. Thus, the progress of the sealing process may be accurately monitored by measuring the transparence of the tissue.

The transparence of the tissue may be measured by focusing light on the tissue and measuring the amount of visible light reflected from or transmitted through the tissue 105. A feedback and control system (e.g., the surgical systems 100, 200 of FIGS. 1 and 2) may monitor changes in the transparency of the tissue and determine when the sealing energy should be removed from the tissue 105 to provide optimum sealing quality.

When optical energy is used for tissue sealing, a negative feedback control loop is established in the tissue being sealed. As the tissue 105 is heated, the tissue's optical transparence increases, thus limiting the amount of optical energy which is absorbed by the tissue 105. The negative feedback control loop helps to avoid charring and to reduce thermal damage of the tissue 105.

Feedback information relating to optical polarization-dependent losses in the tissue and anisotropy of the tissue may also be monitored and used to control the sealing process. The anisotropic structure of collagen molecules results in strong linear birefringence of tissue. See Johannes F. de Boer, Shyam M. Srinivas, Arash Malekafzali, Zhongping Chen and J. Stuart Nelson, "Imaging thermally damaged tissue by polarization sensitive optical coherence tomography," OPTICS EXPRESS, Vol. 3, No. 6, pp. 212-218, 1998). Birefringence of collagen is reduced by the denaturizing that occurs at a temperature between 56°C and 65°C. A corresponding drop in polarization-dependent losses enables direct control of collagen and elastin conditions to ensure high-quality sealing. In particular, the measurement of polarization-dependent losses or anisotropy of the tissue allows one to determine when and what part of the collagen is melted during sealing.

Changes in the optical properties of the tissue 105 during the sealing process may also be related to heat-induced changes in the blood, in particular, to the conversion of oxyhemoglobin and de-oxyhemoglobin in the normal blood to methemoglobin. See U.S. Patent No. 6,766,187. Depending on the wavelength of the light, the absorptivity of methemoglobin can be higher or lower than the absorptivity of normal blood. Therefore, in some embodiments, the wavelength of the light emitted from the light source 112 is selected to be in the range between 525 nm and 580 nm to minimize the influence of heat-induced modification of the optical properties of the blood on the optical feedback signal.

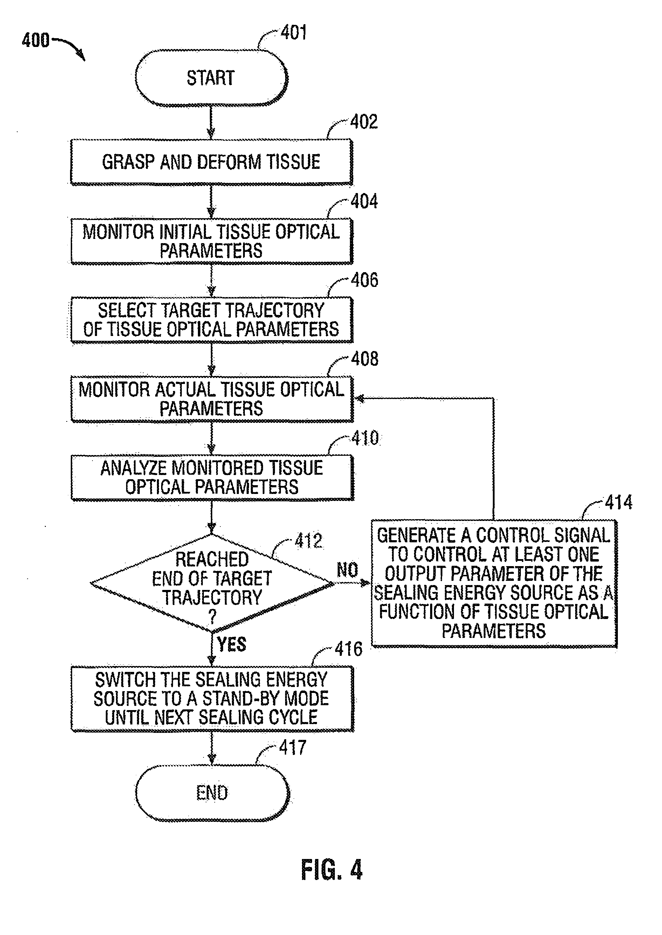

FIG. 4 is a flow diagram 400 of a method of performing energy-based tissue sealing employing optical feedback signals. After starting in step 401, the energy-based instrument 101 includes a mechanism (e.g., jaw members) that grasps and deforms the tissue 105, in step 402. In step 404, the initial optical parameters of the tissue are measured. In step 406, a target trajectory of the optical parameters is selected based on the measured initial optical parameters. The energy-based surgical system varies the parameters of the sealing energy applied to the tissue 105 so that the monitored optical parameters follow or track the selected trajectory of the optical parameters during the sealing procedure. This allows the energy-based surgical system to take into account different properties of different types of tissues that are being sealed and to optimize the parameters of the sealing energy to a particular tissue type.

After a target trajectory of optical parameters is selected, the energy-based surgical system iterates through a series of steps to track the target trajectory of optical parameters: in step 408, the actual optical parameters of tissue are measured or monitored; in step 410, the monitored optical parameters are analyzed with respect to the target trajectory; in step 412, it is determined whether the end of the target trajectory has been reached; and, if the end of the target trajectory has not been reached, in step 414, a control signal is generated to modify the parameters of the output from sealing energy source 102 so that the monitored optical parameters (e.g., the optical signal(s) 118) follow the target trajectory.

When the end of the target trajectory is reached, the sealing energy source 102 is switched into a standby mode until the next tissue sealing cycle. In some embodiments, when the sealing energy source 102 is switched into a standby mode, the monitoring of optical parameters of the tissue is stopped. In other embodiments, however, the monitoring of the optical parameters is continued to control other functions of the energy-based surgical system including to control cooling of the sealed tissue.

The method of FIG. 4 is one of many possible methods of performing tissue sealing based on optical parameters of the tissue. The method may be simplified by omitting the step of selecting a target optical parameter trajectory and using the same target trajectory for all types of tissues. The method may also be simplified by substituting the target optical parameter trajectory with a predetermined optical parameter threshold value. If the measured optical parameter of the tissue reaches the predetermined optical parameter threshold value, the energy-based instrument stops applying sealing energy to the tissue 105.

Although the present disclosure has been described with respect to particular embodiments, it will be readily apparent to those having ordinary skill in the art to which it appertains that changes and modifications may be made thereto without departing from the scope of the disclosure. For example, the controller 103 may include circuitry and other hardware, rather than, or in combination with, programmable instructions executed by a microprocessor for processing the sensed values and determining the control signals to be sent to the power supply 211 and the energy output stage 202.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

## Claims

1. An energy-based tissue-sealing system, comprising:
an energy-based instrument having a mechanism to deform tissue in the form of a vessel and an energy applicator to apply tissue-sealing energy to the tissue;
a sealing energy source (102) coupled to the energy-based instrument and configured to generate and deliver tissue-sealing energy to the energy-based instrument;
wherein the tissue-sealing energy is light and the sealing energy source is a light source;
a tissue monitor configured to monitor at least one optical parameter of the tissue structure during sealing;
wherein the tissue monitor includes a light source (112) configured to illuminate the tissue with light, a sensor configured to sense the light modified by the tissue structure, and a processor configured to determine the at least one optical parameter of the tissue structure based on the light modified by the tissue structure ;
a controller coupled to the sealing energy source and configured to control at least one parameter of the tissue-sealing energy generated by the sealing energy source based on the at least one optical parameter of the tissue structure, wherein:
the light source (112) includes a light element (303) and a beam formation system (304), wherein the light element (303) is configured to generate light, and the beam formation system (304) is configured to form a light beam (305) so that the tissue (105) that is subject to the effects of the sealing energy is exposed to the light beam (305), wherein an angle θ (315) is the angle of an incident light beam (305) with respect to an axis (320) perpendicular to the vessel (302), wherein the beam formation system (304) is configured to vary the angle θ (315) of the light beam (305) over a range of angles, and wherein the beam formation system (304) is configured to select the angle θ (315) of the light beam (305) so as to optimize the detection of the at least one optical parameter of the tissue (105).

2. The energy-based tissue-sealing system of claim 1, wherein the at least one optical parameter of the tissue structure includes one or more of optical transparency of the tissue structure, degree of reflection from the tissue structure, optical losses in the tissue structure caused by absorption or scattering by the tissue structure, polarization-dependent losses in the tissue structure, and degree of anisotropy of the tissue structure.

3. The energy-based tissue-sealing system of claim 1 or 2, wherein the wavelength of the light is between 525 nm and 585 nm.

4. The energy-based tissue-sealing system of claim 1, 2 or 3, comprising
a pair of jaw members providing the mechanism to deform tissue; and wherein the light source is configured to illuminate at least a portion of the tissue deformed by the pair of jaw members with light.

5. The energy-based tissue sealing system of any preceding claim, wherein the sealing energy source is configured to generate light to perform a sealing procedure and a portion of the same light is used for the tissue monitor to monitor the at least one optical parameter.

6. The energy-based tissue sealing system of any preceding claim, wherein the sealing energy source comprises a light-energy output stage and the energy applicator includes at least one optical element, wherein the light-energy output stage is adapted to provide light to the at least one optical element configured to direct or focus the light on tissue.

7. The energy-based tissue sealing system of any preceding claim, comprising a first optical sensor (214a) configured to sense light reflected from the tissue (105) and a second optical sensor (214b) configured to sense light transmitted through the tissue (105).

8. The tissue sealing system of claims 1 to 3 or 5 to 7, wherein the mechanism comprises jaw members (321, 322) for deforming tissue (105) by grasping and compressing the vessel (302) under a predetermined pressure, wherein the jaw members (321, 322) are configured to bring opposite walls of the vessel (302) into contact with each other.

9. The energy-based tissue sealing system of any preceding claim, comprising a first optical system (307) for collecting and a first photo-sensor (308) for detecting light transmitted through the tissue, wherein the tissue monitor is configured to determine optical parameters of the tissue (105) by measuring and analyzing parameters of the light (306) transmitted through the tissue (105).

10. The energy-based tissue sealing system of any preceding claim, comprising a second optical system (310) for collecting and a second photo-sensor (311) for detecting light reflected or scattered from the tissue, wherein the tissue monitor is configured to determine optical parameters of the tissue (105) by measuring and analyzing parameters of the reflected or scattered light (309).

11. The energy-based tissue sealing system of any preceding claim, wherein the system is configured to measure initial optical parameters of the tissue and select a target trajectory of the optical parameters based on the measured initial optical parameters, wherein the energy-based tissue sealing system is configured to vary the parameters of the sealing energy applied to the tissue (105) so that the monitored optical parameters follow or track the selected trajectory of the optical parameters during the sealing procedure, so that the energy-based tissue sealing system is able to take into account different properties of different types of tissues being sealed and to optimize the parameters of the sealing energy to a particular tissue type.

12. The energy-based tissue sealing system of claim 11, wherein, after a target trajectory of optical parameters is selected, the energy-based tissue sealing system is configured to iterate through a step to track the target trajectory of optical parameters as follows:
to measure or monitor the actual optical parameters of tissue;
to analyze the monitored optical parameters with respect to the target trajectory;
to determine whether the end of the target trajectory has been reached; and,
if the end of the target trajectory has not been reached, to generate a control signal to modify the parameters of the output from the sealing energy source (102) so that the monitored optical parameters follow the target trajectory.

13. The energy-based tissue sealing system of any preceding claim, wherein the beam formation system comprises a collimator for forming the incident light beam by generating parallel rays of light.

## Patentansprüche

1. Energiebasiertes Gewebeversiegelungssystem, umfassend:
ein energiebasiertes Instrument mit einem Mechanismus zum Verformen von Gewebe in Form eines Gefäßes und einen Energieapplikator zum Anwenden von Gewebeversiegelungsenergie auf das Gewebe;
eine Versiegelungsenergiequelle (102), die mit dem energiebasierten Gerät gekoppelt und konfiguriert ist zur Erzeugung und Abgabe von Gewebeversiegelungsenergie an das energiebasierte Instrument;
wobei die Gewebeversiegelungsenergie Licht und die Versiegelungsenergiequelle eine Lichtquelle ist;
einen Gewebemonitor, der konfiguriert ist, um mindestens einen optischen Parameter der Gewebestruktur während der Versiegelung zu überwachen;
wobei der Gewebemonitor eine Lichtquelle (112), die konfiguriert ist, das Gewebe mit Licht zu beleuchten; einen Sensor, der konfiguriert ist, das durch die Gewebestruktur modifizierte Licht zu erfassen, und einen Prozessor aufweist, der konfiguriert ist, den mindestens einen optischen Parameter der Gewebestruktur auf der Basis des durch die Gewebestruktur modifizierten Lichts zu bestimmen;
eine Steuereinrichtung, die mit der Versiegelungsenergiequelle gekoppelt ist, um den mindestens einen optischen Parameter der Gewebeversiegelungsenergie zu steuern, der durch die Versiegelungsenergiequelle auf der Basis des mindestens einen optischen Parameters der Gewebestruktur erzeugt wurde, wobei:
die Lichtquelle (112) ein Lichtelement (303) und ein Strahlformungssystem (304) aufweist, wobei das Lichtelement (303) konfiguriert ist, Licht zu erzeugen und das Strahlformungssystem (304) konfiguriert ist,
einen Lichtstrahl (305) derart zu bilden, dass das Gewebe (105), das den Wirkungen der Versiegelungsenergie unterworfen ist, dem Lichtstrahl (305) ausgesetzt ist, wobei ein Winkel θ (315) der Winkel eines einfallenden Lichtstrahls (305) in Bezug auf eine Achse (320) senkrecht zu dem Gefäß (302) ist,
wobei das Strahlformungssystem (304) konfiguriert ist, den Winkel θ (315) des Lichtstrahls (305) über einen Bereich von Winkeln zu variieren und wobei das Strahlformungssystem (304) konfiguriert ist, den Winkel θ (315) des Lichtstrahls (305) derart zu wählen, dass die Erfassung des mindestens einen optischen Parameters des Gewebes (105) optimiert wird.

2. Energiebasiertes Gewebeversiegelungssystem nach Anspruch 1, wobei der mindestens eine optische Parameter eines oder mehrere von der optischen Durchlässigkeit der Gewebestruktur, dem Grad der Reflexion von der Gewebestruktur, der optischen Verluste in der Gewebestruktur, die durch Absorption oder Streuung durch die Gewebestruktur verursacht werden, den polarisationsabhängigen Verlusten in der Gewebestruktur und dem Grad der Anisotropie von der Gewebestruktur umfasst.

3. Energiebasiertes Gewebeversiegelungssystem nach Anspruch 1 oder 2, wobei die Wellenlänge des Lichts zwischen 525 nm und 585 nm liegt.

4. Energiebasiertes Gewebeversiegelungs-Instrument nach Anspruch 1, 2 oder 3, das ein Paar Klemmbackenelemente aufweist, die konfiguriert sind, um Gewebe zu verformen; und wobei die Lichtquelle konfiguriert ist, um mindestens einen Abschnitt des Gewebes mit Licht zu beleuchten, der durch das Paar Klemmbackenelemente verformt wird.

5. Energiebasiertes Gewebeversiegelungssystem nach einem der vorhergehenden Ansprüche, wobei die Versiegelungsenergiequelle konfiguriert ist, Licht zur Durchführung eines Versiegelungsverfahrens zu erzeugen und ein Teil des gleichen Lichts für den Gewebemonitor zur Überwachung des mindestens einen optischen Parameters verwendet wird.

6. Energiebasiertes Gewebeversiegelungssystem nach einem der vorhergehenden Ansprüche, wobei die Versiegelungsenergiequelle eine Lichtenergie-Ausgangsstufe aufweist und der Energieapplikator mindestens ein optisches Element umfasst, wobei die Lichtenergie-Ausgangsstufe eingerichtet ist, Licht zu dem mindestens einen optischen Element zu liefern, das konfiguriert ist, das Licht auf das Gewebe zu lenken oder zu fokussieren.

7. Energiebasiertes Gewebeversiegelungssystem nach einem der vorhergehenden Ansprüche, das einen ersten optischen Sensor (214a) aufweist, der konfiguriert ist, das Licht zu erfassen, welches von dem Gewebe (105) reflektiert wird und der zweite optische Sensor (214b) konfiguriert ist, das Licht zu erfassen, welches durch das Gewebe (105) durchgelassen wird.

8. Gewebeversiegelungssystem nach Anspruch 1 bis 3 oder 5 bis 7, wobei der Mechanismus Klemmbackenelemente (321, 322) zum Verformen von Gewebe (105) durch Ergreifen und Zusammendrücken des Gefäßes (302) unter einem vorbestimmten Druck aufweist, wobei die Klemmbackenelemente (321, 322) konfiguriert sind, gegenüberliegende Wänden des Gefäßes (302) miteinander in Kontakt zu bringen.

9. Energiebasiertes Gewebeversiegelungssystem nach einem der vorhergehenden Ansprüche, das ein erstes optisches System (307) zum Sammeln und einen ersten Photosensor (308) zum Erfassen des durch das Gewebe durchgelassenen Lichts aufweist, wobei der Gewebemonitor konfiguriert ist, die optischen Parameter des Gewebes (105) durch Messen und Analysieren von Parametern des Lichts (306), das durch das Gewebe (105) durchgelassen wurde, zu bestimmen.

10. Energiebasiertes Gewebeversiegelungssystem nach einem der vorhergehenden Ansprüche, das ein zweites optisches System (310) zum Sammeln und einen zweiten Photosensor (311) zum Erfassen des durch das Gewebe reflektierten oder gestreuten Lichts aufweist, wobei der Gewebemonitor konfiguriert ist, die optischen Parameter des Gewebes (105) durch Messen und Analysieren von Parametern des reflektierten oder gestreuten Lichts (309) zu bestimmen.

11. Energiebasiertes Gewebeversiegelungssystem nach einem der vorhergehenden Ansprüche, wobei das System konfiguriert ist, die anfänglichen optischen Parameter des Gewebes zu messen und eine Zieltrajektorie der optischen Parameter anhand der gemessenen optischen Anfangsparameter auszuwählen, wobei das energiebasierte Gewebeversiegelungssystem konfiguriert ist, die Parameter des Versiegelungsenergie, die an das Gewebe (105) angelegt wird, derart zu variieren, dass die überwachten optischen Parameter der ausgewählten Trajektorie der optischen Parameter während des Versiegelungsvorganges folgen oder sie verfolgen, sodass dies es dem energiebasierten Gewebeversiegelungssystem ermöglicht, die unterschiedlichen Eigenschaften der verschiedenen Arten von Gewebe, die versiegelt werden, zu berücksichtigen und die Parameter der Versiegelungsenergie auf einen bestimmten Gewebetyp zu optimieren.

12. Energiebasiertes Gewebeversiegelungssystem nach Anspruch 11, wobei, nachdem eine Zieltrajektorie optischer Parameter ausgewählt ist, das energiebasierte Gewebeversiegelungssystem konfiguriert ist, einen Schritt zu durchlaufen, um die Zieltrajektorie der optischen Parameter wie folgt zu verfolgen:
Messen oder Überwachen der derzeitigen optischen Parameter des Gewebes;
Analysieren der überwachten optischen Parameter in Bezug auf die Zieltrajektorie;
Bestimmen, ob das Ende der Zieltrajektorie erreicht worden ist; und
falls das Ende der Zieltrajektorie nicht erreicht worden ist, ein Steuersignal zu erzeugen, um die Parameter der Ausgabe der Versiegelungsenergiequelle (102) zu modifizieren, sodass die überwachten Parameter der Zieltrajektorie folgen.

13. Energiebasiertes Gewebeversiegelungssystem nach einem der vorhergehenden Ansprüche, wobei das Strahlformungssystem einen Kollimator aufweist, der den einfallenden Lichtstrahl durch Erzeugen paralleler Lichtstrahlen bildet.

## Revendications

1. Système de scellage tissulaire à base d'énergie, comprenant :
un instrument à base d'énergie ayant un mécanisme pour déformer le tissu sous la forme d'un vaisseau et un applicateur d'énergie pour lui appliquer de l'énergie de scellage tissulaire ;
une source d'énergie de scellage (102) couplée à l'instrument à base d'énergie et configurée pour générer et délivrer de l'énergie de scellage tissulaire à l'instrument à base d'énergie ;
dans lequel l'énergie de scellage tissulaire est de la lumière et la source d'énergie de scellage est une source de lumière ;
un contrôleur de tissu configuré pour contrôler au moins un paramètre optique de la structure tissulaire au cours du scellage ;
dans lequel le moniteur de tissu comprend une source de lumière (112) configurée pour éclairer le tissu par la lumière, un capteur configuré pour détecter la lumière modifiée par la structure tissulaire et un processeur configuré pour déterminer le au moins un paramètre optique de la structure tissulaire sur la base de la lumière modifiée par la structure tissulaire ;
un contrôleur couplé à la source d'énergie de scellage et configuré pour commander au moins un paramètre de l'énergie de scellage tissulaire générée par la source d'énergie de scellage sur la base du au moins un paramètre optique de la structure tissulaire, dans lequel :
la source de lumière (112) comprend un élément lumineux (303) et un système de formation de faisceau (304), dans lequel l'élément lumineux (303) est configuré pour générer de la lumière et le système de formation de faisceau (304) est configuré pour former un faisceau lumineux (305) de sorte que le tissu (105) qui est soumis aux effets de l'énergie de scellage soit exposé au faisceau lumineux (305), dans lequel l'angle θ (315) est l'angle d'un faisceau lumineux incident (305) par rapport à un axe (320) perpendiculaire au vaisseau (302) dans lequel le système de formation de faisceau (304) est configuré pour faire varier l'angle θ (315) du faisceau lumineux (305) sur une plage d'angles et dans lequel le système de formation de faisceau (304) est configuré pour sélectionner l'angle θ (315) du faisceau lumineux (305) de manière à optimiser la détection du au moins un paramètre optique du tissu (105).

2. Système de scellage tissulaire à base d'énergie selon la revendication 1, dans lequel le au moins un paramètre optique de la structure tissulaire comprend un(e) ou plus de la transparence optique de la structure tissulaire, du degré de réflexion de la structure tissulaire, des pertes optiques dans la structure tissulaire provoquées par l'absorption ou la dispersion par la structure tissulaire, des pertes en fonction de la polarisation dans la structure tissulaire et du degré d'anisotropie de la structure tissulaire.

3. Système de scellage tissulaire à base d'énergie selon la revendication 1 ou la revendication 2, dans lequel la longueur d'onde de la lumière se situe entre 525 nm et 585 nm.

4. Système de scellage tissulaire à base d'énergie selon la revendication 1, 2 ou 3, comprenant :
une paire d'éléments de mors qui fournit le mécanisme permettant de déformer le tissu ; et dans lequel la source de lumière est configurée pour éclairer au moins une partie du tissu déformé par la paire d'éléments de mors par la lumière.

5. Système de scellage tissulaire à base d'énergie selon l'une quelconque des revendications précédentes, dans lequel la source d'énergie de scellage est configurée pour générer de la lumière afin d'effectuer une procédure de scellage et une partie de la même lumière est utilisée pour le moniteur de tissu afin de surveiller le au moins un paramètre optique.

6. Système de scellage tissulaire à base d'énergie selon l'une quelconque des revendications précédentes, dans lequel la source d'énergie de scellage comprend un étage de sortie d'énergie lumineuse et l'applicateur d'énergie comprend au moins un élément optique, dans lequel l'étage de sortie d'énergie lumineuse est à même de fournir de la lumière au au moins un élément optique configuré pour diriger ou focaliser la lumière sur le tissu.

7. Système de scellage tissulaire à base d'énergie selon l'une quelconque des revendications précédentes, comprenant un premier capteur optique (214a) configuré pour détecter la lumière réfléchie par le tissu (105) et un second capteur optique (214b) configuré pour détecter la lumière transmise à travers le tissu (105).

8. Système de scellage tissulaire selon les revendications 1 à 3 ou 5 à 7, dans lequel le mécanisme comprend des éléments de mors (321, 322) pour déformer le tissu (105) en saisissant et en comprimant le vaisseau (302) sous une pression prédéterminée, dans lequel les éléments de mors (321, 322) sont configurés pour amener les parois opposées du vaisseau (302) en contact l'une avec l'autre.

9. Système de scellage tissulaire à base d'énergie selon l'une quelconque des revendications précédentes, comprenant un premier système optique (307) pour la collecte et un premier photocapteur (308) pour détecter la lumière transmise à travers le tissu, dans lequel le moniteur de tissu est configuré pour déterminer des paramètres optiques du tissu (105) en mesurant et en analysant les paramètres de la lumière (306) transmise à travers le tissu (105).

10. Système de scellage tissulaire à base d'énergie selon l'une quelconque des revendications précédentes, comprenant un second système optique (310) pour la collecte et un second photocapteur (311) pour détecter la lumière réfléchie ou dispersée par le tissu, dans lequel le moniteur de tissu est configuré pour déterminer des paramètres optiques du tissu (105) en mesurant et en analysant des paramètres de la lumière réfléchie ou dispersée (309).

11. Système de scellage tissulaire à base d'énergie selon l'une quelconque des revendications précédentes, dans lequel le système est configuré pour mesurer des paramètres optiques initiaux du tissu et sélectionner une trajectoire cible des paramètres optiques sur la base des paramètres optiques initiaux mesurés, dans lequel le système de scellage tissulaire à base d'énergie est configuré pour modifier les paramètres de l'énergie de scellage appliquée au tissu (105) de sorte que les paramètres optiques surveillés suivent ou pistent la trajectoire sélectionnée des paramètres optiques au cours de la procédure de scellage, de sorte que le système de scellage tissulaire à base d'énergie soit à même de prendre en compte différentes propriétés de différents types de tissus qui sont scellés et d'optimiser les paramètres de l'énergie de scellage appliquée à un type de tissu particulier.

12. Système de scellage tissulaire à base d'énergie selon la revendication 11, dans lequel, après avoir choisi une trajectoire cible de paramètres optiques, le système de scellage tissulaire à base d'énergie est configuré pour repasser par une étape afin de pister la trajectoire cible de paramètres optiques comme suit :
mesurer ou surveiller les paramètres optiques réels du tissu ;
analyser les paramètres optiques surveillés par rapport à la trajectoire cible ;
déterminer si la fin de la trajectoire cible a été atteinte ; et,
si l'extrémité de la trajectoire cible n'a pas été atteinte, générer un signal de commande pour modifier les paramètres de la sortie de la source d'énergie de scellage (102) de façon que les paramètres optiques surveillés suivent la trajectoire cible.

13. Système de scellage tissulaire à base d'énergie selon l'une quelconque des revendications précédentes, dans lequel le système de formation de faisceau comprend un collimateur pour former le faisceau de lumière incident en générant des rayons de lumière parallèles.
